# EUROPEAN PATENT SPECIFICATION

(11) **EP 3 890 803 B1**
(45) Date of publication and mention of the grant of the patent: **18.11.2026**
(21) Application number: 18815634.3
(22) Date of filing: 05.12.2018
(51) Int. Cl.: A61M 1/36

(54) **HEART LUNG MACHINE CONNECTION SYSTEM**
VERBINDUNGSSYSTEM FÜR HERZ-LUNGEN-MASCHINE
SYSTÈME DE CONNEXION DE MACHINE CARDIOPULMONAIRE

(43) Date of publication of application: 13.10.2021
(60) Divisional application: 26211058.8
(73) Proprietor: LivaNova Deutschland GmbH, 80939 Munich (DE)
(72) Inventor: WOLFGRAMM, Olivier, 81479 Munich (DE); BONCZAR, Michael, 84539 Ampfing (DE)
(74) Representative: Grünecker Patent- und Rechtsanwälte PartG mbB
(86) International application number: PCT/EP2018/083662
(87) International publication number: WO 2020/114591

(56) References cited:
- WO-A1-00/47266
- WO-A1-2005/065744
- WO-A2-2016/180950
- US-A- 6 105 912
- US-A1- 2002 009 386
- US-A1- 2003 135 152

## Description

### TECHNICAL FIELD

The present disclosure relates to connection system for medical equipment, in particular, component connection systems for heart lung machines.

### BACKGROUND

Heart lung machines often have components connectable to masts that are coupled to the base. Connecting HLM components to an HLM base and/or a mast often involves a number of separate steps including making the physical connection, connecting cables and tubes to aspects of the HLM, and/or the like.
WO 00/47266 A1 discloses a self-contained pack assembly including all of the disposable components of an extra-corporeal support circuit for cardiac bypass surgery. The pack assembly comprises a blood reservoir, a blood oxygenator, a carrier for mounting the blood reservoir, and blood oxygenator. WO 2005/065744 A1 discloses a disposable, integrated extracorporeal blood circuit employed during cardiopulmonary bypass surgery that performs gas exchange, heat transfer, and microemboli filtering functions in a way to conserve volume, to reduce setup and change out times, to eliminate a venous blood reservoir, and to substantially reduce blood-air interface. Blood from the patient or prime solution is routed through an air removal device that is equipped with air sensors for detection of air. US 2002/009386 A1 discloses a self-contained pack assembly includes all of the disposable components of an extracorporeal support circuit for cardiac bypass surgery. The pack assembly comprises a blood reservoir, a blood oxygenator, and a carrier for vertically mounting the blood reservoir and blood oxygenator. Trays may be releasably attached to the carrier to serve as containers for coiled tubing used in the support circuit. WO 2016/180950 A1 discloses a mount interface that includes a latch; and a latching assembly configured to receive the latch so as to lock the latch at a set position with respect to the latching assembly, wherein the latching assembly includes a housing provided with an irregular orifice configured to receive the latch; a first plug disposed in the housing so that a first portion of the irregular orifice overlies the first plug; and a second plug disposed in the housing adjacent the first plug so that a second portion of the irregular orifice overlies the second plug. US 6 105 912 A discloses a blood reservoir mounting bracket supports alternate sizes of blood reservoir assemblies. The reservoir mounting bracket has a reversible reservoir mounting frame, with mounting pins on one side thereof arranged in a first pattern for the mounting holes of one size of a blood reservoir assembly, and mounting pins on its other side arranged in a second pattern for the mounting holes of a second, different sized blood reservoir assembly. US 2003/135152 A1 discloses a a disposable cartridge for use in an extracorporeal blood perfusion system having a cardiopulmonary circuit for receiving venous blood from a patient, oxygenating the blood and returning the oxygenated blood to the patient, a cardioplegia circuit for delivering a cardioplegia solution to the patient, and a suction circuit for withdrawing blood or fluids from the patient or surgical site, the perfusion system having a control unit for controlling the flow of fluids in one or more of the circuits, the disposable cartridge comprising a housing defining a plurality of internal passageways, a first internal passageway being configured for connection to the cardiopulmonary circuit, a second internal passageway being configured for connection to the cardioplegia circuit and a third internal passageway being configured for connection to the suction circuit.

### SUMMARY

It is provided a connection assembly configured to facilitate connecting at least one heart lung machine, HLM, component to an HLM base, the connection assembly comprising:
a mounting bracket comprising a connection interface configured to facilitate coupling the connection assembly to at least one of the HLM base and a mast of the HLM, wherein the mast is coupled to the HLM base;
a component connector configured to facilitate removably connecting an HLM component to the HLM base and comprising an engagement mechanism; and
a component support, the component support comprising:
   a component mount configured to be coupled to the HLM component;
   a support arm coupled, at a first end, to the component mount, and extending away from the component mount, wherein a second end of the support arm is configured to be releasably engaged by the engagement mechanism of the component connector; and
   one or more illuminating elements configured to illuminate at least a portion of the HLM component.

Further, it is provided a method of preparing a heart lung machine (HLM) for use, the HLM comprising an HLM base; a control assembly connected to the HLM base; and a connection assembly coupled to at least one of the HLM base and a mast that is coupled to the HLM base; the connection assembly comprising a mounting bracket having a connection interface configured to facilitate coupling the connection assembly to the at least one of the HLM base and the mast that is coupled to the HLM base; and a component connector configured to facilitate removably connecting an HLM component to the base, the method comprising:
coupling a component support to an HLM component by attaching the HLM component to a component mount of the component support, wherein the component support includes a support arm coupled, at a first end, to the component mount, and extending away from the component mount;
routing a connection cable through a cable-routing feature of the support arm, wherein the connection cable is connected, at a first end, to the HLM component and includes, at a second end, a cable connector;
coupling the component support to the component connector by engaging a second end of the support arm using an engagement mechanism of the component connector,
wherein coupling the component support to the component connector causes the cable connector to engage a connection element of the component connector; and
providing a user input to a user interface presented by the control assembly, wherein the user input facilitates configuring the connection assembly.

While multiple embodiments are disclosed, still other embodiments of the presently disclosed subject matter will become apparent to those skilled in the art from the following detailed description, which shows and describes illustrative embodiments of the disclosed subject matter. Accordingly, the drawings and detailed description are to be regarded as illustrative in nature and not restrictive.

### BRIEF DESCRIPTION OF THE DRAWINGS

FIG. 1A is a front perspective view of an illustrative heart lung machine (HLM), in accordance with embodiments of the subject matter disclosed herein.
FIG. 1B is a side perspective view of the illustrative HLM depicted in FIG. 1A, in accordance with embodiments of the subject matter disclosed herein.
FIG. 1C is a partial perspective view of the base of the trolley depicted in FIGS. 1A and 1B, in accordance with embodiments of the subject matter disclosed herein.
FIG. 2A is a top perspective view of an illustrative connection assembly, in accordance with embodiments of the subject matter disclosed herein.
FIG. 2B is a top perspective view of another illustrative connection assembly, in accordance with embodiments of the subject matter disclosed herein.
FIG. 3 is a side view of an illustrative connection assembly, in accordance with embodiments of the subject matter disclosed herein.
FIG. 4A is a top view of an illustrative connection assembly, showing an illustrative implementation in accordance with embodiments of the subject matter disclosed herein.
FIG. 4B is a top view of another illustrative connection assembly, showing an illustrative implementation in accordance with embodiments of the subject matter disclosed herein.
FIG. 5 depicts an illustrative sensor hub, in accordance with embodiments of the subject matter disclosed herein.
FIG. 6 depicts an illustrative oxygenator system coupled to a first component support and a second component support, in accordance with embodiments of the subject matter disclosed herein.
FIG. 7 is a perspective view of another illustrative component support, in accordance with embodiments of the subject matter disclosed herein.

While the disclosed subject matter is amenable to various modifications and alternative forms, specific embodiments have been shown by way of example in the drawings and are described in detail below. The intention, however, is not to limit the subject matter disclosed herein to the particular embodiments described. On the contrary, the disclosure is intended to cover all modifications and alternatives falling within the scope of the subject matter defined by the appended claims.

As used herein in association with values (e.g., terms of magnitude, measurement, and/or other degrees of qualitative and/or quantitative observations that are used herein with respect to characteristics (e.g., dimensions, measurements, attributes, components, etc.) and/or ranges thereof, of tangible things (e.g., products, inventory, etc.) and/or intangible things (e.g., data, electronic representations of currency, accounts, information, portions of things (e.g., percentages, fractions), calculations, data models, dynamic system models, algorithms, parameters, etc.), "about" and "approximately" may be used, interchangeably, to refer to a value, configuration, orientation, and/or other characteristic that is equal to (or the same as) the stated value, configuration, orientation, and/or other characteristic or equal to (or the same as) a value, configuration, orientation, and/or other characteristic that is reasonably close to the stated value, configuration, orientation, and/or other characteristic, but that may differ by a reasonably small amount such as will be understood, and readily ascertained, by individuals having ordinary skill in the relevant arts to be attributable to measurement error; differences in measurement and/or manufacturing equipment calibration; human error in reading and/or setting measurements; adjustments made to optimize performance and/or structural parameters in view of other measurements (e.g., measurements associated with other things); particular implementation scenarios; imprecise adjustment and/or manipulation of things, settings, and/or measurements by a person, a computing device, and/or a machine; system tolerances; control loops; machine-learning; foreseeable variations (e.g., statistically insignificant variations, chaotic variations, system and/or model instabilities, etc.); preferences; and/or the like.

The terms "up," "upper," and "upward," and variations thereof, are used throughout this disclosure for the sole purpose of clarity of description and are only intended to refer to a relative direction (i.e., a certain direction that is to be distinguished from another direction), and are not meant to be interpreted to mean an absolute direction. Similarly, the terms "down," "lower," and "downward," and variations thereof, are used throughout this disclosure for the sole purpose of clarity of description and are only intended to refer to a relative direction that is at least approximately opposite a direction referred to by one or more of the terms "up," "upper," and "upward," and variations thereof.

Although the term "block" may be used herein to connote different elements illustratively employed, the term should not be interpreted as implying any requirement of, or particular order among or between, various blocks disclosed herein. Similarly, although illustrative methods may be represented by one or more drawings (e.g., flow diagrams, communication flows, etc.), the drawings should not be interpreted as implying any requirement of, or particular order among or between, various steps disclosed herein. However, certain embodiments may require certain steps and/or certain orders between certain steps, as may be explicitly described herein and/or as may be understood from the nature of the steps themselves (e.g., the performance of some steps may depend on the outcome of a previous step). Additionally, a "set," "subset," or "group" of items (e.g., inputs, algorithms, data values, etc.) may include one or more items, and, similarly, a subset or subgroup of items may include one or more items. A "plurality" means more than one.

### DETAILED DESCRIPTION

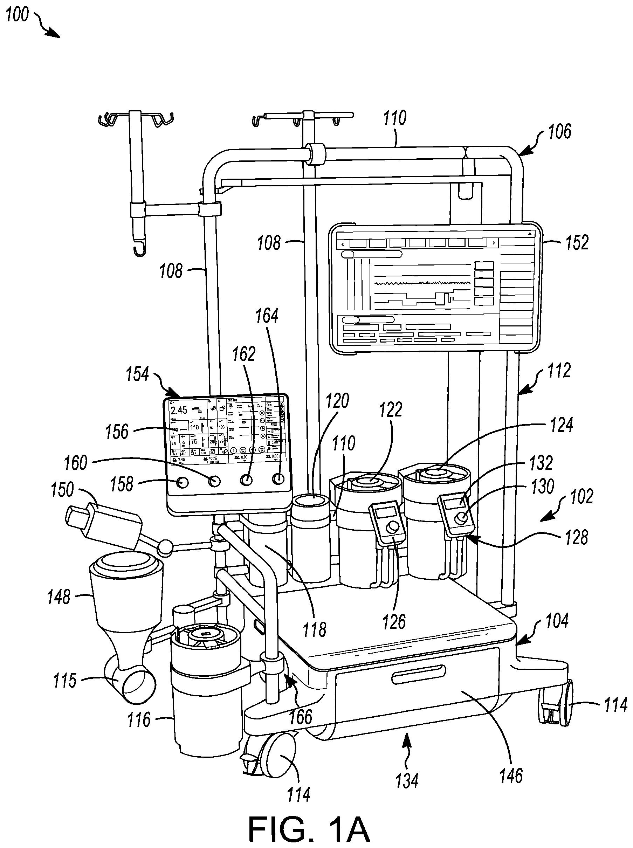

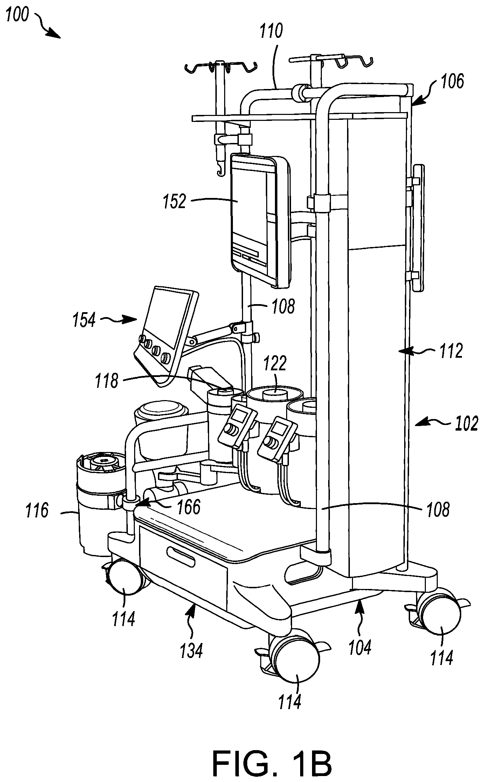

FIG. 1A is a front perspective view of an illustrative heart lung machine (HLM) 100, in accordance with embodiments of the subject matter disclosed herein; and FIG. 1B is a side perspective view of the illustrative HLM 100 depicted in FIG. 1A, in accordance with embodiments of the subject matter disclosed herein. Embodiments of the subject matter described herein may be implemented within the context of any number of different types of HLMs. That is, for example, embodiments of the subject matter may include HLMs having one or more of the features of the illustrative HLM 100. In embodiments, the HLM may include any number of different configurations.

As shown, the HLM 100 includes a trolley 102 having a base 104 that includes an internal cavity (not shown) for housing any number of different controls, electrical circuits, hydraulic circuits, a battery discharger, and/or the like. For example, in embodiments, a peripheral processing unit may be disposed within the base 104. A mast assembly 106 is coupled to the base 104 and extends upwards from the base 104. The mast assembly 106 may include any number of different mast components, including vertical poles 108, horizontal rails 110, and/or the like. In embodiments, the trolley 102 includes an enclsoure 112 that is configured to facilitate cable management, provide A/C outlets, include a power switch for the HLM 100, include an extension box, and/or the like. As shown, the trolley 102 also may include wheels 114 coupled to the base 104.

As shown, the HLM 100 also may include a number of different types of components such as an oxygenator 115 (which may actually be considered to be an element of an extracorporeal circuit used with the HLM, but may be referred to herein as being a component of the HLM due to being connected to the trolley 102); pumps 116, 118, 120, 122, 124; and/or the like. In embodiments, one or more of the components 115, 116, 118, 120, 122, and 124 (and/or others) may be coupled to any number of different portions of the mast assembly 106, and may include, for example, an exposed actuator control unit (ACU). For example, as shown, pumps 122 and 124 may each include an exposed ACU 126 and 128, operably connected thereto, respectively. As shown, an ACU 128 may include a control knob 130 configured to receive user input (e.g., manipulation of the knob 130) for controlling operation of the pump 124, and an information display device 132 configured to present information associated with the pump such as, for example, one or more parameters (e.g., measured device parameters such as, for instance, flow, rpm, etc.). According to embodiments, an ACU may be configured to facilitate control of a pump, a motorized clamp, a motorized occluder, an infusion device, and/or any number of other types of devices that may be associated with an HLM.

Traditionally, HLMs have utilized roller pumps that are each integrated into a modular console component. The modular console components are stacked next to one another on the base of an HLM to provide an array of pumps. The modular console component also houses an ACU having an interface for controlling the corresponding integrated roller pump. One advantage of having the ACU interface provided at the modular console component is that, during an emergency situation, the perfusionist can easily determine the ACU that corresponds to a particular roller pump. More recently, mast mounted roller pumps (without the modular console component housing) have been utilized in HLMs. Mast mounted pumps provide more flexibility in the configuration of the HLM; however, if the ACUs for the mast mounted pumps are located remotely, or detached from, the mast mounted pumps, it's potentially more difficult for the perfusionist to identify the ACU that controls a particular pump.

Embodiments of the present disclosure include mast mounted roller pumps, such as pumps 122 and 124 of FIG. 1A, having corresponding ACUs, such as ACUs 126 and 128, respectively. The ACUs 126 and 128 are attached, connected, or otherwise operatively coupled to the corresponding mast mounted roller pumps 122 and 124. The connectedness, or close proximity of the ACU to the mast mounted roller pump allows the user to precisely determine the ACU that controls a particular mast mounted roller pump in a high pressure, or emergency situation, including a situation where the control display device 156 is not functioning properly or is disabled. Thus, the ability to mount the pumps 122 and 124 to the mast assembly 106 allows a much wider range of configurations to meet the user's particular needs.

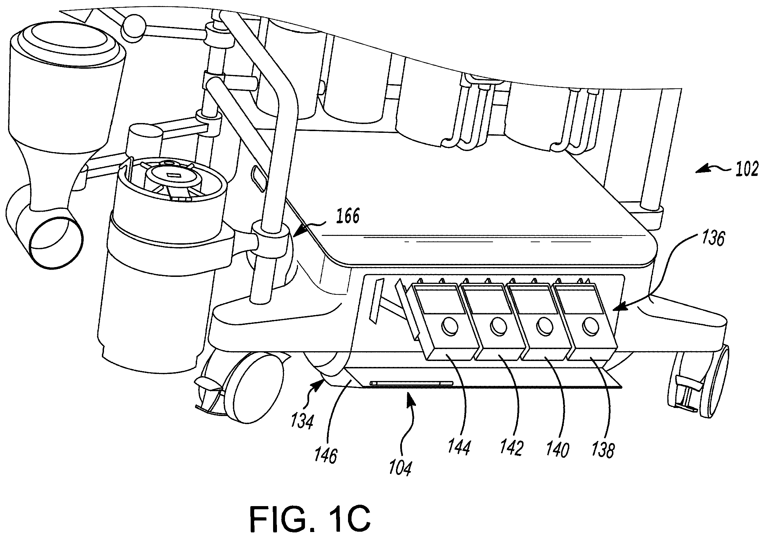

FIG. 1C is a partial perspective view of the base 104 of the trolley 102 depicted in FIGS. 1A and 1B, in accordance with embodiments of the subject matter disclosed herein. As shown in FIG. 1C, the base 104 of the trolley 102 may include a lower housing 134 having an enclosure 136 configured to house one or more ACUs 138, 140, 142, and 144. In embodiments, any number of ACUs may be disposed in the enclosure 136. For example, in embodiments, all of the ACUs for actuators associated with the HLM 100 may be disposed at least partly in the enclosure 136. In embodiments, one or more ACUs may be exposed by being disposed directly on or near the corresponding actuators. According to embodiments, the enclosure 136 may be configured to be closed to protect the ACUs disposed therein, or opened to reveal the ACUs. For example, the lower housing 134 may include a drawer, cabinet, and/or the like. As shown, in embodiments, the lower housing 134 may include a door 146 configured to be opened and closed to selectively expose or conceal the enclosure 136. Each of the ACUs 138, 140, 142, and 144 may be operably connected to a corresponding one of the components 116, 118, 120, 122 (e.g., in cases in which the pump 122 does not include an exposed ACU 126), or 124 (e.g., in cases in which the pump 124 does not include an exposed ACU 128) and/or other actuators.

As is further shown in FIGS. 1A and 1B, the HLM 100 may include any number of other components such as, for example, a venous reservoir 148 (which may be, for example, a component of an extracorporeal circuit that may be used in combination with the HLM 100), an electronic venous occluder (EVO) 150, a peripheral display device 152, a control assembly 154, any number of various types of sensors, and/or the like. According to embodiments, any number of the components discussed herein, others not discussed herein, or aspects of the components (e.g., sensors and/or actuators associated with components) may be operably connected to the peripheral processing unit (not shown), which may be configured to receive parameter data from any one or more of the components, process parameter data, receive control signals from any one or more input devices (e.g., ACU control knobs 130, etc.), provide control signals to any one or more of the components, and/or the like.

In embodiments, the peripheral display device 152 may be operably connected to the peripheral processing unit and configured to present a set of parameter data received from the peripheral processing unit. In embodiments, the peripheral display device 152 may be, include, or be included within a data recording and/or management system. That is, for example, the peripheral display device 152 may include, or be otherwise associated with, a processing unit separate from that of the HLM, and/or may be configured to record and/or display any number of different operative HLM parameters. In some implementations, for example, the peripheral display device 152 may be configured to obtain and record all of the operative HLM parameter values and/or patient parameters provided by any number of additional monitoring devices. The peripheral display device 152 may be configured to present, graphically, representations of any number of the obtained parameter values, changes in parameter values over time, derived parameter values (e.g., values derived from parameter values), and/or the like.

During an operation, the primary focus of a user of the HLM 100 generally is the oxygenator 115 and the venous reservoir 148. Accordingly, embodiments of the subject matter disclosed herein provide a control assembly 154 near those two components 115 and 148 so that the user can access control devices and view displayed parameters without having to move away from, or be distracted from, the oxygenator 115 and venous reservoir 148. According to embodiments, the control assembly 154 may include a control display device 156 and a number of input control devices 158, 160, 162, and 164. In embodiments, the control assembly 154 may include any number of input control devices (e.g., 1, 2, 3, 4, 5, 6, etc.) and the number of input control devices may be less than or equal to the number of ACUs in the enclosure 136. The control display device 156 may be configured to present a subset of the parameters presented by the peripheral display device 152 and/or the peripheral display device 152 may be configured to present a subset of the subset of parameters presented by the control display device 156. A different subset of the set of parameter data may be displayed by the peripheral display device 152. In embodiments, the peripheral display device 152 may be configured to display real-time waveform traces, while the control display device 156 may be configured to display numerical representations of the same and/or different parameters.

That is, for example, regardless of what is displayed on the peripheral display device 152, the control display device 156 may be configured to display a specified subset of parameter data that is particularly useful and/or important with respect to a procedure being performed. That specified subset of parameter data may be predetermined, based on the type of procedure; dynamically presented, based on a status of the patient and/or device; and/or the like. In embodiments, all of the information configured to be presented on the control display device 156 may be presented simultaneously - that is, without having tabs for accessing screens showing additional information, without requiring menus for accessing screens showing additional information during a procedure, and/or the like. In embodiments, the control display device 156 may include selectable representations presented onscreen that can be used to configure the display such as, for example, by enabling a user to select a display mode corresponding to a particular HLM component (e.g., a centrifugal pump, a roller pump, etc.), to select a particular display module (e.g., a pre-configured set of data fields in a particular arrangement), and/or the like.

According to embodiments, the peripheral display device 152 and/or the control display device 156 may include an input mechanism configured to enable user interaction with one or more features displayed on the display device 152 and/or 156. That is, for example, the peripheral display device 152 and/or the control display device 156 may be, or include, a touchscreen device configured to receive user input. In embodiments, the peripheral display device 152 and/or the control display device 156 may include an input device connected thereto such as, for example, a mouse, a trackpad, a joystick, and/or the like.

According to embodiments, for example, additional data from devices external to the HLM (e.g., blood gas monitors, electrocardiographs, ventilators, patient monitors, etc.) may be displayed on the peripheral display device 152. As indicated above, the peripheral display device 152 may be controlled by a peripheral processing unit that is separate from the central system unit of the HLM. The peripheral processing unit associated with the peripheral display device 152 may be configured to obtain parameter values (e.g., from the central system unit, sensors, actuators, external devices, etc.) and may be configured to collect the data in a database. The peripheral processing unit may be communicatively coupled to the peripheral display device 152, HLM components, and/or external devices. In embodiments, while the peripheral processing unit may be configured to receive data from the central system unit, the peripheral processing unit may be configured so as to not send any data to the central system unit. In other embodiments, the peripheral processing unit and the central system unit may be configured to exchange data with one another and/or other devices. According to embodiments, a user may select which data is to be stored by which processing or system unit.

The peripheral processing unit associated with the peripheral display device may be configured to allow user interaction therewith, generate reports based on the obtained data, generate printable documents corresponding to a medical procedure, interact with a printer to cause the printer to print such reports, and/or the like. In embodiments, the peripheral processing unit may be configured to generate, and cause the peripheral display device to present, graphs (e.g., trend charts, curves, etc.) and/or other visual representations of any number of various aspects of data received from HLM components and/or external devices. In embodiments the peripheral display device may be configurable such that a user can select certain types of data and/or representations thereof to display, the manner in which it is displayed, and/or the like. In contrast, for example, the control display device 156 may include only limited configurability, if at all. In this manner, the control display device 156 can be relied upon to present representations of data relevant to the HLM's current use. According to other embodiments, the control display device 156 may have any amount of configurability.

In embodiments, each of the input control devices 158, 160, 162, and 164 may be operably connected to one of the actuators and may be configured to receive user input for controlling an operation of the actuator. According to embodiments, the input control devices 158, 160, 162, and 164 may be operably connected to the respective ACUs 138, 140, 142, and 144, in which case, the input control devices 158, 160, 162, and 164 act in parallel to the ACUs, but do not have priority over them in controlling the actuators. In embodiments, the input control devices are directly connected to the respective ACUs, and the ACUs are connected to the respective actuators, such that an actuator can be controlled by an input control device only through an ACU or directly by an ACU. Therefore, the ACU has prevalence over the input control device in controlling the actuator.

As is further shown in FIGS. 1A and 1B (and described in more detail below), the HLM 100 may include a connection assembly 166 configured to facilitate connecting at least one HLM component to the HLM base 104. The connection assembly 166 may include any number of different connection elements configured to facilitate operatively connecting an HLM component to other components of the HLM. In embodiments, the connection elements may include fluid connection elements, energy connection elements, and/or data connection elements. In embodiments, the control assembly 154 may be configured to facilitate configuring the connection assembly 166. According to embodiments, configuring the connection assembly 166 may include, for example, providing input to a control unit via the control assembly 154 that assigns a particular type of HLM component to the connection assembly 166 and/or a connection element thereof, assigns a particular HLM component to the connection assembly 166 and/or a connection element thereof, causes the control assembly to display a representation of the connection element and/or HLM component, causes the control assembly to include data received via the connection element to be displayed in a user interface, and/or the like.

According to embodiments, any one or more of the components of the illustrative HLM 100 may be implemented on one or more computing devices. A computing device may include any type of computing device suitable for implementing aspects of embodiments of the disclosed subject matter. Examples of computing devices include specialized computing devices or general-purpose computing devices such "control units," "control assemblies," "workstations," "servers," "hand-held devices," "heart lung machines," "controllers," and the like, all of which are contemplated within the scope of FIG. 1, with reference to various components of the HLM 100.

In embodiments, a computing device includes a bus that, directly and/or indirectly, couples the following devices: a processing unit, a memory, an input/output (I/O) port, an I/O component, and a power supply. Any number of additional components, different components, and/or combinations of components may also be included in the computing device. The I/O component may include a presentation component configured to present information to a user such as, for example, a display device, a speaker, a printing device, and/or the like, and/or an input component such as, for example, a microphone, a joystick, a satellite dish, a scanner, a printer, a wireless device, a keyboard, a pen, a voice input device, a touch input device, a touch-screen device, an interactive display device, a mouse, and/or the like.

The bus represents what may be one or more busses (such as, for example, an address bus, data bus, or combination thereof). Similarly, in embodiments, the computing device may include a number of processing units, a number of memory components, a number of I/O ports, a number of I/O components, and/or a number of power supplies. Additionally any number of these components, or combinations thereof, may be distributed and/or duplicated across a number of computing devices.

In embodiments, the memory includes computer-readable media in the form of volatile and/or nonvolatile memory and may be removable, nonremovable, or a combination thereof. Media examples include Random Access Memory (RAM); Read Only Memory (ROM); Electronically Erasable Programmable Read Only Memory (EEPROM); flash memory; optical or holographic media; magnetic cassettes, magnetic tape, magnetic disk storage or other magnetic storage devices; data transmissions; and/or any other medium that can be used to store information and can be accessed by a computing device such as, for example, quantum state memory, and/or the like. In embodiments, the memory stores computer-executable instructions for causing the processor to implement aspects of embodiments of system components discussed herein and/or to perform aspects of embodiments of methods and procedures discussed herein.

The computer-executable instructions may include, for example, computer code, machine-useable instructions, and the like such as, for example, program components capable of being executed by one or more processors associated with the computing device. Program components may be programmed using any number of different programming environments, including various languages, development kits, frameworks, and/or the like. Some or all of the functionality contemplated herein may also, or alternatively, be implemented in hardware and/or firmware.

The illustrative HLM 100 shown in FIGS. 1A - 1C is not intended to suggest any limitation as to the scope of use or functionality of embodiments of the present disclosure. The illustrative HLM 100 also should not be interpreted as having any dependency or requirement related to any single component or combination of components illustrated therein. Additionally, various components depicted in FIGS. 1A - 1C may be, in embodiments, integrated with various ones of the other components depicted therein (and/or components not illustrated), all of which are considered to be within the ambit of the present disclosure.

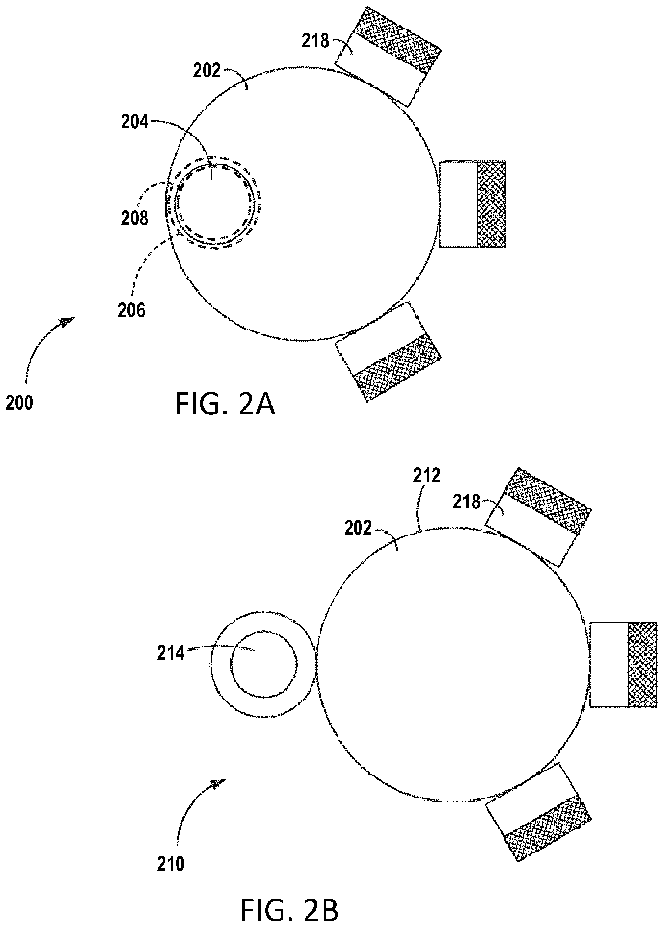

FIG. 2A is a top perspective view of an illustrative connection assembly 200, in accordance with embodiments of the subject matter disclosed herein. FIG. 2B is a top perspective view of another illustrative connection assembly 210, in accordance with embodiments of the subject matter disclosed herein. According to embodiments, the illustrative connection assembly 200 and/or 210 is configured to facilitate connecting at least one heart lung machine (HLM) component to an HLM base, and may be, be similar to, include, or be included within the connection assembly 166 depicted in FIGS. 1A - 1B.

As shown in FIG. 2A, for example, the illustrative connection assembly 200 includes a mounting bracket 202 that may be generally shaped as a cylinder (e.g., a disc). In embodiments, the mounting bracket 202 may have any number of other shapes, and/or may include multiple shaped portions. The mounting bracket 202 includes a connection interface configured to facilitate coupling the connection assembly to the HLM base and/or a mast of the HLM, which may be coupled to the HLM base. In embodiments, for example, the connection assembly 200 may include a swiveling mast insert 204 configured to be disposed within an aperture 206 defined through the mounting bracket 202. The swiveling mast insert 204 may be configured to rotatably couple the mounting bracket 202 to a mast 208 of a medical device such as, for example, an HLM (e.g., the HLM 100 depicted in FIGS. 1A-1B). In embodiments, the connection assembly 200 may additionally, or alternatively, include a mast holder such as, for example, a quick-connect mast holder disposed on an outer surface of the mounting bracket configured for coupling the mounting bracket to the mast. A quick-connect mast holder may be designed to be connected and disconnected from a mast with relative ease (e.g., by performing one or two steps). According to embodiments, a connection interface configured to facilitate coupling the connection assembly to the HLM base may include any number of different types of connection mechanisms, quick-connect mechanisms, and/or the like.

As shown in FIG. 2B, for example, the connection assembly 210 may include an outer surface 212 and a quick-connect mast holder 214 may be attached (removably or permanently) to the outer surface 212. The mast holder 214 may be designed according to any number of different mast-connection designs and may be configured to removably couple the mounting bracket 202 to a mast (not shown) of a medical device such as, for example, an HLM (e.g., the HLM 100 depicted in FIGS. 1A-1B). In embodiments, the connection assembly 210 may include a quick-connect mast holder 214 and a swiveling mast insert (e.g., the swiveling mast insert 204 depicted in FIG. 2A).

With continued reference to FIG. 2A, the connection assembly 200 may also include one or more component connectors 218. Each component connector 218 is configured to facilitate removably coupling an HLM component to the connection assembly 200 and, thus, to the HLM base. The HLM component may include, for example, a pump assembly, an actuator, a sensor assembly, a reservoir, a vacuum system, a control assembly, a display device, a sensor hub (which may include, for example, a number of sensor connection elements), and/or the like. In embodiments, the connection assembly 200 may include a number of component connectors 218 so that a number of HLM components can be connected to the HLM base via the connection assembly 200.

The illustrative connection assemblies 200 and 210 shown in FIGS. 2A -2B are not intended to suggest any limitation as to the scope of use or functionality of embodiments of the present disclosure. The illustrative connection assemblies 200 and 210 also should not be interpreted as having any dependency or requirement related to any single component or combination of components illustrated therein. Additionally, various components depicted in FIGS. 2A - 2B may be, in embodiments, integrated with various ones of the other components depicted therein (and/or components not illustrated), all of which are considered to be within the ambit of the present disclosure.

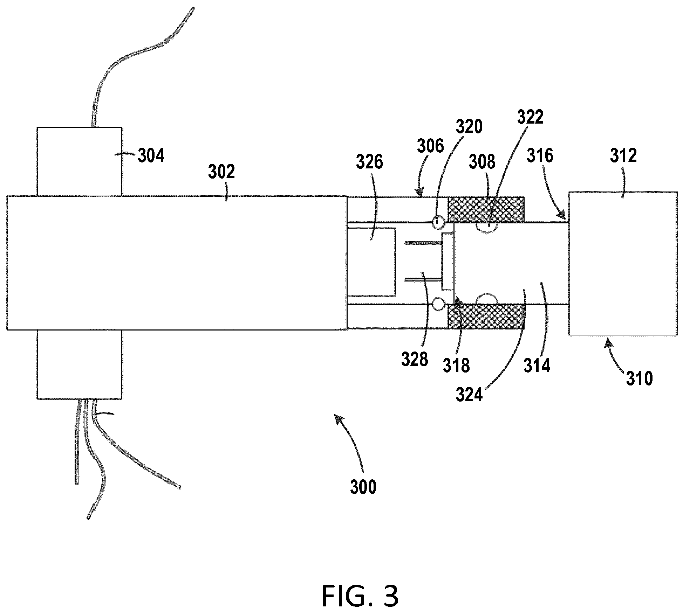

FIG. 3 is a side view of an illustrative connection assembly 300, in accordance with embodiments of the subject matter disclosed herein. The connection assembly 300 may be, be similar to, include, or be included in, the connection assembly 166 depicted in FIGS. 1A-1B, the connection assembly 200 depicted in FIG. 2A, and/or the connection assembly 210 depicted in FIG. 2B. In embodiments, the connection assembly 300 includes a mounting bracket 302 comprising a connection interface 304 configured to facilitate coupling the connection assembly 300 to an HLM base and/or a mast of the HLM. As shown, for example, the mast interface 304 may include a swiveling mast insert configured to route cables into an interior lumen of the mast.

As shown in FIG. 3, the connection assembly 300 may further include a component connector 306 configured to facilitate removably coupling an HLM component to the mounting bracket 302 and, thus, to the HLM base. The component connector includes an engagement mechanism 308 configured to engage a portion of an HLM component and/or a component support 310. In embodiments, an HLM component may include a portion such as, for example, an extended arm, a protrusion, and/or the like, that may be configured to be engaged by (e.g., received in a manner so as to be removably retained by) the engagement mechanism 308. As shown in FIG. 3, in embodiments, an HLM component may be coupled to the HLM via a component support 310.

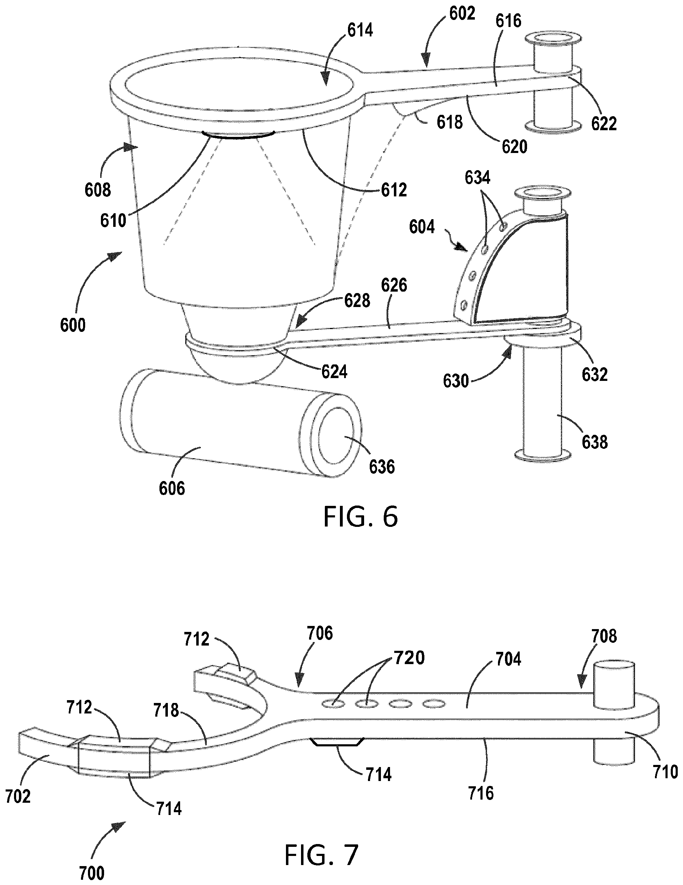

The component support 310, as shown, may include, for example, a component mount 312 configured to be coupled to an HLM component and a support arm 314 coupled, at a first end 316, to the component mount 312, and extending away from the component mount 312. A second end 318 of the support arm 314 may be configured to be releasably engaged by the engagement mechanism 308 of the component connector 306. The engagement mechanism 308 may be configured to removably engage an HLM component and/or a component support 310 using any number of different types of engagement techniques. According to embodiments (and as shown, for example, in FIGS. 6 and 7), the component support 310 may also include one or more illuminating elements configured to illuminate at least a portion of the HLM component to which the component support 310 is connected and/or one or more support connection elements (e.g., fluid connection elements, data connection elements, energy connection elements, etc.).

In embodiments, for example, the engagement mechanism 308 may include a clamp, an interference fit interface, a ball-detent mechanism, and/or the like. As shown in FIG. 3, for example, the engagement mechanism 308 may include a ball-detent mechanism having one or more spring-biased balls 320 configured to be partially received by corresponding detents 322 defined in the support arm 314 of the component support 310 (or an aspect of an HLM component configured to be engaged by the engagement mechanism 308). In the illustrated example, the component support 310 is configured to be removably coupled to the connection assembly 300 by sliding the second end 318 of the support arm 314 into the component connector 306. An outside surface 324 of the support arm 314 displaces the one or more balls 320 as the support arm 314 slides into the component connector 306, until the one or more balls 320 are aligned with the corresponding detents 322, causing the one or more balls 320 to be pushed partially into the corresponding detents 322 by the biasing spring (not shown), thereby holding the support arm 314 in place within the component connector 306. The support arm 314 may be released from the component connector 306 by pulling the support arm 314 out from the component connector 306 with a force sufficient to cause the outside surface 324 of the support arm 314 to displace the one or more balls 320.

The connection assembly 300 also may include one or more connection elements 326 such as, for example, a fluid connection element, an energy connection element, and/or a data connection element. In embodiments, a connection element 326 may include, for example, a male or female aspect of a tube connection, a cable connection, and/or the like, and may include any number of different types of connectors, couplings, quick disconnect couplings, plugs, and/or the like. A connection element 326 may be configured to engage a component connection element 328, which may be a connection element extending from the second end 318 of the support arm 314 of the component support 310 or from an end of a protrusion extending from a component. In embodiments, the connection element 326 and/or the component connection element 328 may be covered (or at least partially covered) by a sleeve (not shown) configured to protect the connection element 326 or component connection element 328.

The connection element 326 and component connection element 328 may be configured to be coupled to one another to facilitate a fluid connection, an energy connection, and/or a data connection. According to embodiments, a fluid connection may include a liquid connection and/or a gas connection. For example, a fluid connection may include a connection between a blood tube disposed within an interior lumen of the HLM mast and an input or output of a blood pump, blood reservoir, and/or the like. A fluid connection may include a connection between a tube that is configured to provide a pharmaceutical solution from a reservoir coupled to the HLM to another HLM component, a connection between a tube that is configured to provide a gas (e.g., oxygen, carbon dioxide, etc.) from a reservoir to another HLM component, and/or the like.

An energy connection may include, for example, a connection between a cable (e.g., one or more wires) at least partially routed through an interior lumen of an HLM mast and an energy input of an HLM component. For example, the connection element 326 may include an electrical socket configured to receive an electrical plug (e.g., the component connection element 328). In embodiments, a data connection may include a connection between a cable at least partially routed through an interior lumen of the HLM mast and a data input of an HLM component. For example, the connection element 326 may include a data socket (e.g., a universal serial bus (USB) port, a serial connection (e.g., an RS-232 connection), an Ethernet port, etc.) configured to receive a data plug (e.g., the component connection element 328).

In embodiments, the connection element 326 may be an energy and data connection. For example, in embodiments, a control assembly of an HLM (e.g., the control assembly 154 depicted in FIGS. 1A and 1B) may be coupled to a peripheral processing unit and/or a bus of the HLM using only one cable. That cable may be configured to transport control signals, parameter data, power, and/or the like. The cable may be, include, or be included within, a bus. For example, the bus may be implemented using circuitry within a central system unit, one or more cables configured to couple the central system unit to one or more different components of the HLM. In embodiments, one or more cables may be used to connect the bus to any number of different HLM components such as, for example, pump actuators, sensors, and/or the like.

The illustrative connection assembly 300 shown in FIG. 3 is not intended to suggest any limitation as to the scope of use or functionality of embodiments of the present disclosure. The illustrative connection assembly 300 also should not be interpreted as having any dependency or requirement related to any single component or combination of components illustrated therein. Additionally, various components depicted in FIG. 3 may be, in embodiments, integrated with various ones of the other components depicted therein (and/or components not illustrated), all of which are considered to be within the ambit of the present disclosure.

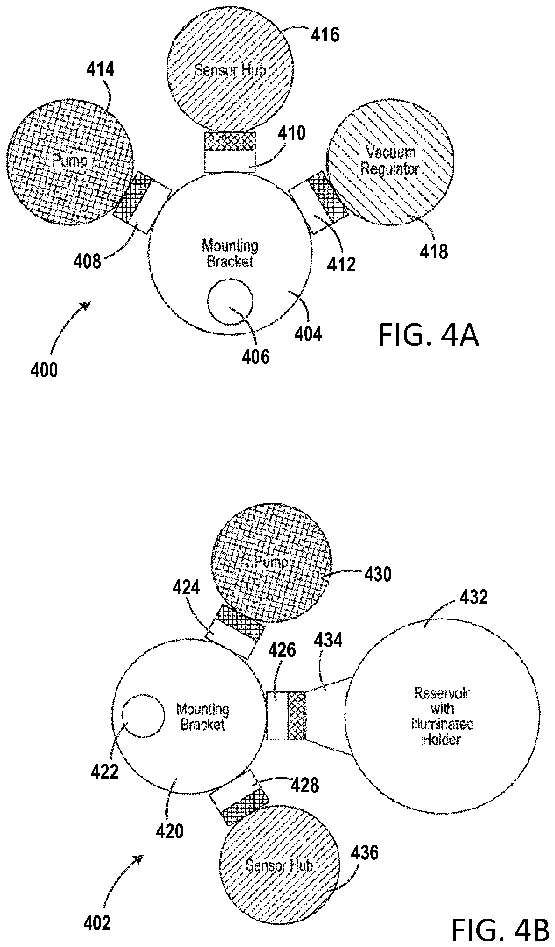

FIG. 4A is a top view of an illustrative connection assembly 400, showing an illustrative implementation in accordance with embodiments of the subject matter disclosed herein; and FIG. 4B is a top view of another illustrative connection assembly 402, showing an illustrative implementation in accordance with embodiments of the subject matter disclosed herein. The connection assembly 400 and/or 402 may be, be similar to, include, or be included in, the connection assembly 166 depicted in FIGS. 1A-1B, the connection assembly 200 depicted in FIG. 2A, the connection assembly 210 depicted in FIG. 2B, and/or the connection assembly 300 depicted in FIG. 3. In embodiments, the connection assembly 402 may be the connection assembly 400, with different HLM components connected thereto.

As shown in FIG. 4A, the connection assembly 400 includes a mounting bracket 404 having a connection interface 406 configured to facilitate coupling the connection assembly 400 to an HLM base and/or a mast of the HLM. The connection assembly 400 includes a first component connector 408, a second component connector 410, and a third component connector 412. Each of the first, second, and third component connectors 408, 410, and 412 is configured to facilitate removably coupling an HLM component to the HLM base. For example, as shown, a pump 414 may be coupled to the first component connector 408, a sensor hub 416 may be coupled to the second component connector 410, and a vacuum regulator 418 may be coupled to the third component connector 412.

Similarly, as shown in FIG. 4B, the connection assembly 402 includes a mounting bracket 420 having a connection interface 422 configured to facilitate coupling the connection assembly 402 to an HLM base and/or a mast of the HLM. The connection assembly 402 includes a first component connector 424, a second component connector 426, and a third component connector 428. Each of the first, second, and third component connectors 424, 426, and 428 is configured to facilitate removably connecting an HLM component to the HLM base. For example, as shown, a pump 430 may be coupled to the first component connector 424, a reservoir 432 may be coupled to the second component connector 426 (e.g., via an illuminated component support 434), and a sensor hub 436 may be coupled to the third component connector 428.

The illustrative connection assemblies 400 and 402 shown in FIGS. 4A -4B are not intended to suggest any limitation as to the scope of use or functionality of embodiments of the present disclosure. The illustrative connection assemblies 400 and 402 also should not be interpreted as having any dependency or requirement related to any single component or combination of components illustrated therein. For example, each of the connection assemblies 400 and 402 may include any number of component connectors. Similarly, any number of different HLM components may be coupled, in any number of different combinations and/or arrangements, to the connection assemblies 400 and 402. Additionally, various components depicted in FIGS. 4A - 4B may be, in embodiments, integrated with various ones of the other components depicted therein (and/or components not illustrated), all of which are considered to be within the ambit of the present disclosure.

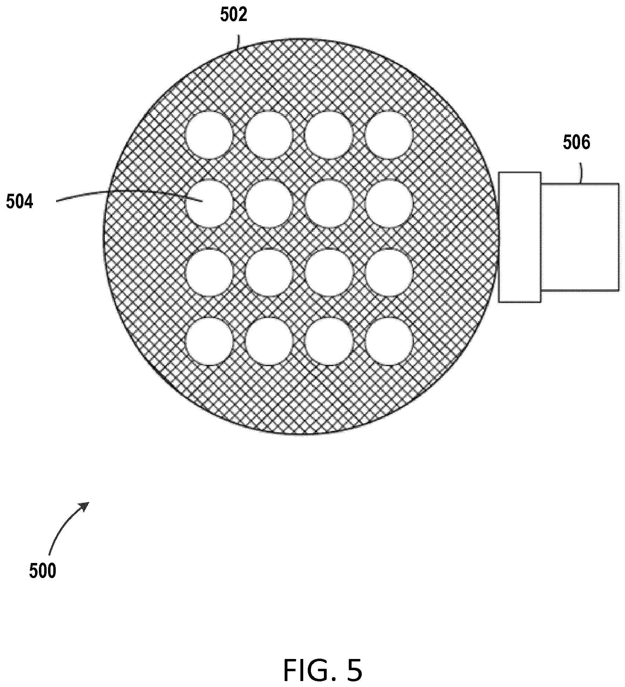

FIG. 5 depicts an illustrative sensor hub 500, in accordance with embodiments of the subject matter disclosed herein. According to embodiments, the sensor hub 500 may be, be similar to, include, or be included within the sensor hub 416 depicted in FIG. 4A and/or the sensor hub 436 depicted in FIG. 4B. As shown, the sensor hub 500 includes a body 502 having a number of hub connection elements 504. The body 502 may be configured to be removably coupled to a component connector 506 of a connection assembly. According to embodiments, the hub connection elements 504 may include connection sockets and/or ports disposed within the body 502. The hub connection elements 504 may include fluid connection elements, energy connection elements, and/or data connection elements. For example, the hub connection elements may include a temperature sensor interface, a pressure sensor interface, a level sensor interface, a bubble sensor interface, and/or the like. Any number of connection elements of any kind may be included in a sensor hub 500, and each connection element may provide a sensor interface and may be associated with a sensor channel (e.g., as categorized by a computing device of the system such as, for example, a control assembly).

In embodiments, as shown, the hub connection elements 504 may be arranged in an array having a number of rows and/or columns. In this manner, each connection element 504 may be assigned an identifier based on its position within the array. In embodiments, the sensor hub 500 also may have its own identifier (e.g., to distinguish it from other sensor hubs connected to the HLM base). In embodiments, the connection elements 504 may be of a single type of connection interface (e.g., universal serial bus (USB) ports, Ethernet ports, RS-232 ports, etc.) or multiple types of connection interfaces. In embodiments, the sensor hub 500 may be configured to provide certain interfaces to an HLM by assigning each hub connection element 504 a specified type of interface. In this manner, all of the hub connection elements of a sensor hub 500 may be used for a single type of sensor interface (e.g., by connecting multiple component connection elements associated with multiple sensors throughout the system to the sensor hub) or multiple types of sensor interfaces (e.g., by connecting all of the sensors associated with an HLM component or components to the sensor hub).

According to embodiments, each connection element 504 and/or group of connection elements 504 may be equipped with (or otherwise associated with) one or more visual signal devices (e.g., light-emitting diodes (LEDs), muli-colored light indicators, etc.) configured to indicate a connection status of the element(s) 504. For example, in embodiments, a multi-colored connection indicator may light up red if there is no connection, yellow if there is a problem with a connection, and green if all connections associated with the element(s) 504 are good. In embodiments, the connection indicator may be configured to blink, illuminate according to any number of various patterns, and/or the like. Embodiments may further include alarms (e.g., audio alarms, haptic alarms, etc.) that can be triggered upon the detection of connection problems.

According to embodiments, the sensor hub 500 may include a control unit (e.g., a microcontroller) that may be configured to perform one or more functions associated with operation of the sensor hub 500. In embodiments, a control unit of the HLM may be configured to perform the one or more functions (or any aspects thereof). According to embodiments, the control unit (e.g., a sensor hub control unit and/or an HLM control unit) may be configured to maintain an assignment of sensor types, devices, protocols, and/or the like corresponding to each of the hub connection elements of the sensor hub 500. That is, for example, a user may be able to interact with a control assembly (e.g., the control assembly 154 depicted in FIGS. 1A and 1B) to assign a particular sensor type to each of the hub connection elements of the sensor hub. In this manner, for example, a user may configure a sensor hub to provide four temperature channels, four pressure channels, two air bubble channels, and one fluid level channel. According to embodiments, the control unit may be configured to determine when a component connection element has been connected to one of the hub connection elements, to identify the type of sensor that has been connected to it, to identify the specific sensor (e.g., where each sensor of an HLM may be uniquely identifiable to facilitate organization of data received therefrom, etc.), and/or the like.

According to embodiments, upon connecting a sensor hub 500 to an HLM, a user may be able to configure, via interaction with a control assembly of the HLM, the hub connection elements 504 of the sensor hub 500, a displayed representation of the sensor hub 500, and/or the like. For example, the control assembly may be configured to present, on a display device (a display device of the control assembly, a central display device, a display device of an external device, and/or the like), a representation of the sensor hub. The representation of the sensor hub may include a representation of each of the hub connection elements of the sensor hub, which may, for example, be presented in an arrangement that corresponds to the arrangement of the hub connection elements.

Each of the representations of the hub connection elements may be interactive. That is, for example, a user may be able to select a hub connection element of the sensor hub by selecting (using an input device such as, e.g., a mouse, a finger, a button, a dial, etc.) the corresponding representation of the hub connection element. Upon selection of the hub connection element, the control assembly may be configured to present, on the display device, any number of different types of information, user interfaces, and/or the like. For example, in embodiments, upon receiving an indication of a user selection of the representation of the hub connection element, the control assembly may be configured to present a user interface that enables a user to configure the hub connection element (e.g., by assigning a particular sensor type), to cause the control assembly to display the representation of the hub connection element, to cause the control assembly to include data received via the hub connection element to be displayed in a user interface, and/or the like. In this manner, for example, when a user connects a sensor hub to an HLM, the user may select which channels are to be displayed, which display device the selected channels are to be displayed on, and/or the like.

In embodiments, when a user connects a sensor hub to an HLM (thereby operatively coupling the sensor hub to a control unit (e.g., a processing unit) of the HLM), the user may be prompted (e.g., via a control assembly) to provide a device identifier corresponding to the sensor hub (e.g., a number or name that identifies the sensor hub). The control unit may be configured to determine whether the provided device identifier is currently in use (assigned to a sensor hub) and, if so, may be configured to inform (via a display device) the user that the device identifier is assigned to a sensor hub and to ask the user whether the user wishes to replace the existing sensor hub to which the device identifier is assigned. Upon receiving a user input indicating that the user wishes to replace the existing sensor hub, the control unit may be configured to apply the configuration of the existing sensor hub to the new sensor hub. In embodiments, the control unit may be configured to apply the existing configuration to the new sensor hub in response to detecting connection of the new sensor hub to the system.

According to embodiments, in response to receiving a user input indicating that the user wishes to add another sensor hub to the system, the control unit may present, on the display device, a representation of each of the hub connection elements of the sensor hub. A user may interact with the representations of the hub connection elements to select which channels provided thereby will be used; where, on a user interface, data obtained via each channel will be displayed; how much data to display from each channel; the format of the data displayed from each channel; and/or the like. In embodiments, one or more of these configurations may be selected by the user by interacting with interactive elements of the representation of the sensor hub, interactive elements of a representation of the patient (e.g., in which identifications of the various sensor types are shown in proximity to locations on the patient from which the corresponding data are obtained. In embodiments, sensor channels that are not used may not have a corresponding location for display of data on the user interface. Any number of different combinations of configurations may be facilitated in embodiments of the subject matter disclosed herein.

The illustrative sensor hub 500 shown in FIG. 5 is not intended to suggest any limitation as to the scope of use or functionality of embodiments of the present disclosure. The illustrative sensor hub 500 also should not be interpreted as having any dependency or requirement related to any single component or combination of components illustrated therein. Additionally, various components depicted in FIG. 5 may be, in embodiments, integrated with various ones of the other components depicted therein (and/or components not illustrated), all of which are considered to be within the ambit of the present disclosure.

FIG. 6 depicts an illustrative oxygenator system 600 coupled to a first component support 602 and a second component support 604, in accordance with embodiments of the subject matter disclosed herein. According to embodiments, the oxygenator system 600 may be, be similar to, include, or be included within the oxygenator 115 and/or venous reservoir 148 (one or more of which may be, for example, a component of an extracorporeal circuit that includes the HLM), depicted in FIGS. 1A and 1B. As shown, the oxygenator system 600 may include an oxygenator 606 coupled to a venous reservoir 608. The first component support 602 may be configured to hold (or at least partially support) the reservoir 608 and the second component support 604 may be configured to provide additional support to the reservoir 608 and/or the oxygenator 606.

As shown in FIG. 6, the first component support 602 may include one or more illuminating elements 610 configured to illuminate at least a portion of an HLM component connected to the first component support 602 (e.g., the oxygenator system 600). In embodiments, for example, the one or more illuminating elements 610 may include a light emitting diode (LED), a fiber-optic outlet, and/or the like. As shown, the illuminating element(s) 610 may be disposed on a lower side 612 of a component mount 614 of the component support 602. In this manner, the illuminating element(s) 610 may be configured to illuminate an interior of the reservoir 608. Additionally, as shown in FIG. 6, a support arm 616 may include an optical level sensor 618 disposed on a lower side 620 thereof. The component support 602 may include any number of other sensors, illuminating elements, and/or the like. The component support 602 may be configured to be coupled to a connection assembly 622. According to embodiments, the connection assembly 622 may be, be similar to, include, or be included within the connection assembly 166 depicted in FIGS. 1A and 1B, the connection assembly 200 depicted in FIG. 2, the connection assembly 300 depicted in FIG. 3, the connection assembly 400 depicted in FIG. 4A, and/or the connection assembly 402 depicted in FIG. 4B.

As is further shown in FIG. 6, the second component support 604 may include a component mount 624 configured to be coupled to the reservoir 608 and a support arm 626 coupled, at a first end 628, to the component mount 624, where a second end 630 of the support arm 626 is configured to be releasably engaged by an engagement mechanism (not shown) of a component connector 632. The component support 604 may include a number of connection elements 634 configured to facilitate removably coupling fluid, energy, and/or data component connections to the HLM. In embodiments, the position of the second component connector 604 allows blood connectors 636 to be at approximately the same height as a nearby pump head. Internal lumens defined within the support arms 616 and 626 may be configured to receive cables, tubes, and/or the like, and route them to a mast 638 for routing to other portions of the HLM.

The illustrative oxygenator 600 and illumination assembly 602 shown in FIG. 6 are not intended to suggest any limitation as to the scope of use or functionality of embodiments of the present disclosure. The illustrative oxygenator 600 and illumination assembly 602 also should not be interpreted as having any dependency or requirement related to any single component or combination of components illustrated therein. Additionally, various components depicted in FIG. 5 may be, in embodiments, integrated with various ones of the other components depicted therein (and/or components not illustrated), all of which are considered to be within the ambit of the present disclosure.

FIG. 7 is a perspective view of another illustrative component support 700, in accordance with embodiments of the subject matter disclosed herein. As shown, the illustrative component support 700 may include a component mount 702 configured to be coupled to an HLM component. A support arm 704, coupled, at a first end 706, extends away from the component mount 702 to a second end 708, which may be configured to be releasably engaged by an engagement mechanism of a component connector. In other embodiments, the component support 700 may include a mast interface 710 disposed at the second end 708 of the support arm 704. As shown, the component support 700 may include one or more integrated illuminating elements 712 disposed on an upper surface 718 of the component support 700. One or more integrated cameras 714 may be disposed on a lower surface 716 of the component support 700, and one or more connection elements 720 may be disposed on the upper surface 718.

The illustrative component support 700 shown in FIG. 7 is not intended to suggest any limitation as to the scope of use or functionality of embodiments of the present disclosure. The illustrative component support 700 also should not be interpreted as having any dependency or requirement related to any single component or combination of components illustrated therein. For example, the component support 700 may include any number of different combinations of sensors, illuminating elements, cameras, and/or the like, positioned in any number of different positions. Additionally, various components depicted in FIG. 7 may be, in embodiments, integrated with various ones of the other components depicted therein (and/or components not illustrated), all of which are considered to be within the ambit of the present disclosure.

As explained above, embodiments of the subject matter described herein include connection assemblies configured to facilitate removably coupling an HLM component to an HLM base. According to embodiments, a method of preparing a heart lung machine (HLM) for use is provided. The HLM may be, be similar to, include, or be included within the HLM 100 depicted in FIGS. 1A and 1B. The HLM may include a base, a mast coupled to the base, and a connection assembly coupled to the mast; the connection assembly including a mounting bracket having a mast interface configured to facilitate coupling the connection assembly to the mast and a component connector configured to facilitate removably coupling an HLM component to the base.

According to embodiments, the method may include coupling a component support to an HLM component by attaching the HLM component to a component mount of the component support, where the component support includes a support arm coupled, at a first end, to the component mount, and extending away from the component mount. The method may further include routing a connection cable through a cable-routing feature of the support arm, where the connection cable is connected, at a first end, to the HLM component and includes, at a second end, a cable connector. In embodiments, the method may include coupling the component support to the component connector by engaging a second end of the support arm using an engagement mechanism of the component connector, where coupling the component support to the component connector causes the cable connector to engage a connection element of the component connector. Any number of other steps may be performed in accordance with embodiments of the method.

Various modifications and additions can be made to the exemplary embodiments discussed without departing from the scope of the present disclosure. For example, while the embodiments described above refer to particular features, the scope of this disclosure also includes embodiments having different combinations of features and embodiments that do not include all of the described features. Accordingly, the scope of the present disclosure is intended to embrace all such alternatives, modifications, and variations as fall within the scope of the claims.

## Claims

1. A connection assembly (200) configured to facilitate connecting at least one heart lung machine, HLM, component to an HLM base (104), the connection assembly comprising:
a mounting bracket (202) comprising a connection interface configured to facilitate coupling the connection assembly to at least one of the HLM base (104) and a mast of the HLM, wherein the mast is coupled to the HLM base (104);
a component connector (218) configured to facilitate removably connecting an HLM component to the HLM base (104) and comprising an engagement mechanism; and
a component support (310), the component support (310) comprising:
a component mount (312) configured to be coupled to the HLM component;
a support arm (314) coupled, at a first end, to the component mount (312), and extending away from the component mount (312), wherein a second end of the support arm (314) is configured to be releasably engaged by the engagement mechanism of the component connector (218); and
one or more illuminating elements (610) configured to illuminate at least a portion of the HLM component.

2. The connection assembly (200) of claim 1, the connection interface comprising a mast interface, the mast interface comprising at least one of a swiveling mast insert and a quick-connect mast holder.

3. The connection assembly (200) of either of claims 1 or 2, the component connector (218) comprising at least one of a fluid connection element, an energy connection element, and a data connection element.

4. The connection assembly (200) of claim 1, the component support (310) further comprising one or more support connection elements, each of the one or more support connection elements, the one or more support connection elements comprising fluid connection elements, data connection elements, and/or energy connection elements.

5. The connection assembly (200) of any of claims 1 and 4, the one or more illuminating elements (610) comprising at least one of a light emitting diode, LED, and a fiber-optic outlet.

6. The connection assembly (200) of any of claims 1-5, further comprising an additional component connector configured to facilitate removably connecting an additional HLM component to the HLM base (104).

7. The connection assembly (200) of any of claims 1-6, the HLM component comprising at least one of a pump, a reservoir, an oxygenator, and a sensor hub.

8. The connection assembly (200) of claim 7, the HLM component comprising a sensor hub, the sensor hub comprising a plurality of hub connection elements, the plurality of hub connection elements comprising fluid connection elements and/or data connection elements.

9. The connection assembly (200) of claim 8, the plurality of connection elements comprising at least one of a temperature sensor interface, a pressure sensor interface, a level sensor interface, and a bubble sensor interface.

10. A heart lung machine, HLM, comprising:
an HLM base (104); and
a connection assembly (200) of any of the claims 1 to 9.

11. A method of preparing a heart lung machine, HLM, for use, the HLM comprising an HLM base; a control assembly connected to the HLM base; and a connection assembly coupled to at least one of the HLM base and a mast that is coupled to the HLM base (104); the connection assembly comprising a mounting bracket (202) having a connection interface configured to facilitate coupling the connection assembly to the at least one of the HLM base (104) and the mast that is coupled to the HLM base (104); and a component connector (218) configured to facilitate removably connecting an HLM component to the HLM base (104), the method comprising:
coupling a component support (310) to an HLM component by attaching the HLM component to a component mount (312) of the component support (310), wherein the component support (310) includes a support arm (314) coupled, at a first end, to the component mount (312), and extending away from the component mount (312);
routing a connection cable through a cable-routing feature of the support arm (314), wherein the connection cable is connected, at a first end, to the HLM component and includes, at a second end, a cable connector;
coupling the component support (310) to the component connector (218) by engaging a second end of the support arm using an engagement mechanism of the component connector (218) , wherein coupling the component support (310) to the component connector (218) causes the cable connector to engage a connection element of the component connector (218); and
providing a user input to a user interface presented by the control assembly, wherein the user input facilitates configuring the connection assembly.

## Patentansprüche

1. Verbindungsanordnung (200), die dazu eingerichtet ist, das Verbinden mindestens einer Komponente einer Herz-Lungen-Maschine, HLM, mit einer HLM-Basis (104) zu erleichtern, wobei die Verbindungsanordnung umfasst:
eine Montagehalterung (202), umfassend eine Verbindungsschnittstelle, die dazu eingerichtet ist, das Koppeln der Verbindungsanordnung an mindestens eines von HLM-Basis (104) und einem Mast der HLM zu erleichtern, wobei der Mast mit der HLM-Basis (104) gekoppelt ist;
einen Komponentenverbinder (218), der dazu eingerichtet ist, das lösbare Verbinden einer HLM-Komponente mit der HLM-Basis (104) zu erleichtern, und einen Eingriffsmechanismus umfasst; und
einen Komponententräger (310), wobei der Komponententräger (310) umfasst:
eine Komponentenaufnahme (312), die dazu eingerichtet ist, mit der HLM-Komponente gekoppelt zu werden;
einen Tragarm (314), der an einem ersten Ende mit der Komponentenaufnahme (312) gekoppelt ist und sich von der Komponentenaufnahme (312) weg erstreckt, wobei ein zweites Ende des Tragarms (314) dazu eingerichtet ist, von dem Eingriffsmechanismus des Komponentenverbinders (218) lösbar in Eingriff gebracht zu werden; und
ein oder mehrere Beleuchtungselemente (610), die dazu eingerichtet sind, mindestens einen Abschnitt der HLM-Komponente zu beleuchten.

2. Verbindungsanordnung (200) nach Anspruch 1, wobei die Verbindungsschnittstelle eine Mastschnittstelle umfasst, wobei die Mastschnittstelle mindestens eines aus einem schwenkbaren Masteinsatz und einem Schnellverbindungs-Masthalter umfasst.

3. Verbindungsanordnung (200) nach einem der Ansprüche 1 oder 2, wobei der Komponentenverbinder (218) mindestens eines aus einem Fluidverbindungselement, einem Energieverbindungselement und einem Datenverbindungselement umfasst.

4. Verbindungsanordnung (200) nach Anspruch 1, wobei der Komponententräger (310) ferner ein oder mehrere Trägerverbindungselemente umfasst, jedes der ein oder mehreren Trägerverbindungselemente, wobei das eine oder die mehreren Trägerverbindungselemente Fluidverbindungselemente, Datenverbindungselemente und/oder Energieverbindungselemente umfassen.

5. Verbindungsanordnung (200) nach einem der Ansprüche 1 und 4, wobei das eine oder die mehreren Beleuchtungselemente (610) mindestens eines aus einer Leuchtdiode, LED, und einem Lichtleitfaserausgang umfassen.

6. Verbindungsanordnung (200) nach einem der Ansprüche 1 bis 5, ferner umfassend einen zusätzlichen Komponentenverbinder, der dazu eingerichtet ist, das lösbare Verbinden einer zusätzlichen HLM-Komponente mit der HLM-Basis **(104)** zu erleichtern.

7. Verbindungsanordnung (200) nach einem der Ansprüche 1 bis 6, wobei die HLM-Komponente mindestens eines aus einer Pumpe, einem Reservoir, einem Oxygenator und einem Sensor-Hub umfasst.

8. Verbindungsanordnung (200) nach Anspruch 7, wobei die HLM-Komponente einen Sensor-Hub umfasst, wobei der Sensor-Hub eine Mehrzahl von Hub-Verbindungselementen umfasst, wobei die Mehrzahl von Hub-Verbindungselementen Fluidverbindungselemente und/oder Datenverbindungselemente umfasst.

9. Verbindungsanordnung (200) nach Anspruch 8, wobei die Mehrzahl von Verbindungselementen mindestens eines aus einer Temperatursensorschnittstelle, einer Drucksensorschnittstelle, einer Füllstandssensorschnittstelle und einer Blasensensorschnittstelle umfasst.

10. Herz-Lungen-Maschine, HLM, umfassend:
eine HLM-Basis **(104);** und
eine Verbindungsanordnung (200) nach einem der Ansprüche 1 bis 9.

11. Verfahren zum Vorbereiten einer Herz-Lungen-Maschine, HLM, zur Verwendung, wobei die HLM eine HLM-Basis; eine mit der HLM-Basis verbundene Steuerungsanordnung; und eine Verbindungsanordnung umfasst, die mit mindestens einem von der HLM-Basis und einem Mast gekoppelt ist, der mit der HLM-Basis (104) gekoppelt ist; wobei die Verbindungsanordnung eine Montagehalterung (202) mit
einer Verbindungsschnittstelle, die dazu eingerichtet ist, das Koppeln der Verbindungsanordnung an mindestens eines von der HLM-Basis (104) und dem Mast, der mit der HLM-Basis (104) gekoppelt ist, zu erleichtern; und einen Komponentenverbinder (218) umfasst, der dazu eingerichtet ist, das lösbare Verbinden einer HLM-Komponente mit der HLM-Basis (104) zu erleichtern, wobei das Verfahren umfasst:
Koppeln eines Komponententrägers (310) mit einer HLM-Komponente durch Anbringen der HLM-Komponente an einer Komponentenaufnahme (312) des Komponententrägers (310), wobei der Komponententräger (310) einen Tragarm (314) aufweist, der an einem ersten Ende mit der Komponentenaufnahme (312) gekoppelt ist und sich von der Komponentenaufnahme (312) weg erstreckt;
Führen eines Verbindungskabels durch eine Kabelführungseinrichtung des Tragarms (314), wobei das Verbindungskabel an einem ersten Ende mit der HLM-Komponente verbunden ist und an einem zweiten Ende einen Kabelverbinder aufweist;
Koppeln des Komponententrägers (310) mit dem Komponentenverbinder (218) durch In-Eingriff-Bringen eines zweiten Endes des Tragarms unter Verwendung eines Eingriffsmechanismus des Komponentenverbinders (218), wobei das Koppeln des Komponententrägers (310) mit dem Komponentenverbinder (218) bewirkt, dass der Kabelverbinder mit einem Verbindungselement des Komponentenverbinders (218) in Eingriff gelangt; und
Bereitstellen einer Benutzereingabe an einer durch die Steuerungsanordnung dargestellten Benutzerschnittstelle, wobei die Benutzereingabe das Konfigurieren der Verbindungsanordnung erleichtert.

## Revendications

1. Ensemble de connexion (200) configuré pour faciliter la connexion d'au moins un composant d'une machine cardiopulmonaire, HLM, à une base de la HLM (104), l'ensemble de connexion comprenant :
un support de montage (202) comprenant une interface de connexion configurée pour faciliter le couplage de l'ensemble de connexion à au moins l'un parmi la base de la HLM (104) et un mât de la HLM, le mât étant couplé à la base de la HLM (104) ;
un connecteur de composant (218) configuré pour faciliter la connexion amovible d'un composant de la HLM à la base de la HLM (104) et comprenant un mécanisme d'engagement ; et
un support de composant (310), le support de composant (310) comprenant :
un organe de montage du composant (312) configuré pour être couplé au composant de la HLM ;
un bras de support (314) couplé, au niveau d'une première extrémité, à l'organe de montage du composant (312), et s'étendant à l'écart de l'organe de montage du composant (312), une seconde extrémité du bras de support (314) étant configurée pour être mise en prise de manière libérable par le mécanisme d'engagement du connecteur de composant (218) ; et
un ou plusieurs éléments d'éclairage (610) configurés pour éclairer au moins une partie du composant de la HLM.

2. Ensemble de connexion (200) selon la revendication 1, l'interface de connexion comprenant une interface de mât, l'interface de mât comprenant au moins l'un parmi un insert de mât pivotant et un support de mât à connexion rapide.

3. Ensemble de connexion (200) selon l'une ou l'autre des revendications 1 ou 2, le connecteur de composant (218) comprenant au moins l'un parmi un élément de connexion de fluide, un élément de connexion d'énergie et un élément de connexion de données.

4. Ensemble de connexion (200) selon la revendication 1, le support de composant (310) comprenant en outre un ou plusieurs éléments de connexion du support, chacun des un ou plusieurs éléments de connexion du support, les un ou plusieurs éléments de connexion du support comprenant des éléments de connexion de fluide, des éléments de connexion de données et/ou des éléments de connexion d'énergie.

5. Ensemble de connexion (200) selon l'une quelconque des revendications 1 et 4, les un ou plusieurs éléments d'éclairage (610) comprenant au moins l'un parmi une diode électroluminescente, LED, et une sortie à fibre optique.

6. Ensemble de connexion (200) selon l'une quelconque des revendications 1 à 5, comprenant en outre un connecteur de composant supplémentaire configuré pour faciliter la connexion amovible d'un composant supplémentaire de la HLM à la base de la HLM (104).

7. Ensemble de connexion (200) selon l'une quelconque des revendications 1 à 6, le composant de la HLM comprenant au moins l'un parmi une pompe, un réservoir, un oxygénateur et un concentrateur de capteurs.

8. Ensemble de connexion (200) selon la revendication 7, le composant de la HLM comprenant un concentrateur de capteurs, le concentrateur de capteurs comprenant une pluralité d'éléments de connexion du concentrateur, la pluralité d'éléments de connexion du concentrateur comprenant des éléments de connexion de fluide et/ou des éléments de connexion de données.

9. Ensemble de connexion (200) selon la revendication 8, la pluralité d'éléments de connexion comprenant au moins l'une parmi une interface de capteur de température, une interface de capteur de pression, une interface de capteur de niveau et une interface de capteur de bulles.

10. Machine cardiopulmonaire, HLM, comprenant :
une base de la HLM **(104)** ; et
un ensemble de connexion (200) selon l'une quelconque des revendications 1 à 9.

11. Procédé de préparation d'une machine cardiopulmonaire, HLM, en vue de son utilisation, la HLM comprenant une base de la HLM ; un ensemble de commande connecté à la base de la HLM ; et un ensemble de connexion couplé à au moins l'un parmi la base de la HLM et un mât qui est couplé à la base de la HLM (104) ; l'ensemble de connexion comprenant un support de montage (202) comportant
une interface de connexion configurée pour faciliter le couplage de l'ensemble de connexion à au moins l'un parmi la base de la HLM (104) et le mât qui est couplé à la base de la HLM **(104)** ; et un connecteur de composant (218) configuré pour faciliter la connexion amovible d'un composant de la HLM à la base de la HLM **(104),** le procédé comprenant :
le couplage d'un support de composant (310) à un composant de la HLM par fixation du composant de la HLM à un organe de montage du composant (312) du support de composant (310), le support de composant (310) comprenant un bras de support (314) couplé, au niveau d'une première extrémité, à l'organe de montage du composant (312), et s'étendant à l'écart de l'organe de montage du composant (312) ;
le cheminement d'un câble de connexion à travers un dispositif de cheminement de câble du bras de support (314), le câble de connexion étant connecté, au niveau d'une première extrémité, au composant de la HLM et comprenant, au niveau d'une seconde extrémité, un connecteur de câble ;
le couplage du support de composant (310) au connecteur de composant (218) par mise en prise d'une seconde extrémité du bras de support à l'aide d'un mécanisme d'engagement du connecteur de composant (218), le couplage du support de composant (310) au connecteur de composant (218) amenant le connecteur de câble à venir en prise avec un élément de connexion du connecteur de composant (218) ; et
la fourniture d'une entrée utilisateur à une interface utilisateur présentée par l'ensemble de commande, l'entrée utilisateur facilitant la configuration de l'ensemble de connexion.
